# EUROPÄISCHE PATENTANMELDUNG

(11) **EP 0 842 953 A2**
(43) Veröffentlichungstag der Anmeldung: **20.05.1998**
(21) Anmeldenummer: 97119017.8
(22) Anmeldetag: 31.10.1997
(51) Int. Cl.: C08F 8/12, A61L 15/00

(54) **Präformierte superabsorber mit hohem Quellvermögen**

(30) Priorität: 13.11.1996 DE 19646856
(71) Anmelder: BAYER AG, 51368 Leverkusen (DE)
(72) Erfinder: Sackmann, Günter, Dr., 51379 Leverkusen (DE); Schapowalow, Sergej, Dr., 51061 Köln (DE); Korte, Siegfried, Dr., 51519 Odenthal (DE); Meyer, Rolf-Volker, Dr., 51373 Leverkusen (DE)

(57) **Zusammenfassung**

Superabsorbierendes Polymerisat in Form präformierter kugelförmiger Teilchen erhältlich durch alkalische Hydrolyse von wäßrigen Emulsionen vernetzter und/oder unvernetzter Homo- und/oder Copolymerisate des Acrylnitrils in Wasser/Alkohol-Gemischen und die Verwendung des Polymerisats in Hygieneprodukten, als wasserspeicherndes Material in der Landwirtschaft und zur Ummantelung von Elektrokabeln.

## Beschreibung

Die Erfindung betrifft die Herstellung von präformierten, kugelförmigen superabsorbierenden Polymerisaten mit ausgezeichnetem Quellungsvermögen.

Superabsorbierende Polymerisate sind bekannt und werden hauptsächlich bei der Herstellung von Windeln und Inkontinenzartikeln, aber auch als wasserspeichernde Materialien in der Landwirtschaft sowie bei der Ummantelung von Elektrokabeln verwendet. In der Regel handelt es sich bei diesen superabsorbierenden Polymerisaten um weitmaschig vernetzte, wasserunlösliche Polymerisate oder Copolymerisate auf Basis von Alkalisalzen der Polyacrylsäure oder Copolymerisate von Alkalisalzen der Acrylsäure und Acrylamid, die durch radikalisch initiierte Copolymerisation von Acrylsäure und polyfunktionellen Monomeren, wie Divinylbenzol, Ethylenglykoldimethacrylat, Ethylenglykoldiallylether, Butandiolacrylat, Hexandiolmethacrylat, Polyglykoldiacrylat, Trimethylolpropandiacrylat, Allylacrylat, Diallylacrylamid, Trisallylamin, Diallylether, Methylenbisacrylamid und N-Methylolacrylamid, erhalten werden. Aufgrund ihrer Struktur sind solche Polymerisate in der Lage, unter Quellung und Ausbildung von Hydrogelen große Mengen an Wasser und wäßrigen Lösungen aufzunehmen und diese auch unter Druck festzuhalten.

In den US-Patenten 5 496 890 und 5 635 565 sowie in der Europäischen Patentanmeldung 0 670 335 und der Deutschen Offenlegungsschrift 44 29 318 sind pulverförmige Polymerisate mit superabsorbierenden Eigenschaften beschrieben, die durch die alkalische Hydrolyse von wäßrigen feinteiligen Emulsionen unvernetzter und/oder vernetzter Homo- und/oder Copolymerisate des Acrylnitrils erhalten werden. Bei diesem Verfahren können die Produkte mit superabsorbierenden Eigenschaften durch Ausfällen mit Lösungsmitteln, wie z.B. aliphatischen Monoalkoholen, als feinteilige Pulver isoliert werden. Diese müssen nach dem Filtrieren und Trocknen durch Mahlen und Sieben auf das für die Anwendung gewünschte Korngrößenspektrum gebracht werden, was einen besonderen Schritt mit erheblichem Zeit- und Energieaufwand bedeutet.

Es in ein Ziel der Erfindung Polymerisate mit superabsorbierenden Eigenschaften, direkt, d.h. ohne Mahlen und Sieben mit der gewünschten Teilchengröße und Teilchengrößenverteilung herzustellen.

Gegenstand der Erfindung sind somit superabsorbierende Polymerisate in Form von präformierten kugelförmigen Teilchen, deren Teilchengrößenverteilung im Bereich von 100 bis 850 µm liegt, wobei der Massenanteil der Teilchen mit einem Durchmesser <100 µm weniger als 10 bis 15 Gew.-% beträgt.

Ein weiterer Gegenstand der Erfindung ist ein Verfahren zur Herstellung von superabsorbierenden Polymerisaten in dieser Teilchenform, worin man feinteilige, wäßrige Emulsionen von vernetzten und/oder unvernetzten Homo- und/oder Copolymerisaten des Acrylnitrils durch Umsetzung mit Alkalihydroxiden in Wasser/Alkohol-Gemischen partiell hydrolysiert und die im Verlauf der Reaktion ausfallenden Pulver abtrennt, trocknet und gegebenenfalls kurz erhitzt.

Als Ausgangsprodukte zur Herstellung der erfindungsgemäßen superabsorbierenden Polymerisate dienen feinteilige, wäßrige Emulsionen von Acrylnitril-Homo- und/oder Copolymerisaten (PAN-Emulsionen), deren Herstellung z.B. in der Europäischen Patentanmeldung 0 590 460 beschrieben ist. Diese Emulsionen können sowohl lineare, unvernetzte Homo- und/oder Copolymerisate mit Molekulargewichten von 5·10⁵ bis 1·10⁷ g/Mol, bevorzugt von 2·10⁶ bis 5·10⁶ g/Mol, als auch vernetzte Homo- und/oder Copolymerisate enthalten. Zur Herstellung dieser Emulsionen mit Feststoffgehalten bis zu 55 Gew.-% können spezielle anionische polymere Emulgatoren eingesetzt werden, die ebenfalls in der Europäischen Patentanmeldung 0 590 460 beschrieben sind.

Die mittels Laserkorrelations-Spektroskopie bestimmten mittleren Teilchendurchmesser der unvernetzten wie auch der vernetzten Emulsionen liegen im Bereich von 100 bis 200 nm. Um Emulsionen zu erhalten, die vernetzte Homo- und/oder Copolymere des Acrylnitrils enthalten, werden bei der Polymerisation polyfunktionelle Monomere, wie z.B. Divinylbenzol, Ethylenglykoldimethacrylat, Trisallylamin u.a., in Mengen von 0,2 bis 4,0 Gew. -%, bezogen auf Acrylnitril, eingesetzt.

Die präformierten, kugelförmigen Polymerisate mit superabsorbierenden Eigenschaften werden durch Umsetzung der feinteiligen Polymerisatemulsionen mit Alkalihydroxiden in Wasser/Alkohol-Gemischen bei 50 bis 100°C erhalten. Das molare Verhältnis der Nitrilgruppen der Ausgangspolymerisate zu den Hydroxylgruppen der Alkalihydroxide ist bevorzugt 1:1 bis 1:0,1, besonders bevorzugt 1:0,8 bis 1:0,5.

Als Alkohole werden primäre, aliphatische Monoalkohole, wie z.B. Methanol, Ethanol, n-Propanol, i-Propanol, n-Butanol oder i-Butanol eingesetzt. Das Gewichtsverhältnis von Wasser zu Alkohol beträgt dabei 10:1 bis 1:10, bevorzugt 5:1 bis 1:5, besonders bevorzugt 2:1 bis 1:2. Durch das Wasser:Alkohol-Verhältnis und die Art des Alkohols können sowohl die mittleren Teilchendurchmesser als auch die Teilchengrößenverteilung gesteuert werden. So erhält man mit zunehmendem Alkoholanteil Produkte mit kleinerem mittlerem Teilchendurchmesser und engerer Teilchengrößenverteilung. Die Wasserlöslichkeit des Alkohols beeinflusst ebenfalls die mittleren Teilchendurchmesser und die Teilchengrößenverteilung. So führen Alkohole mit sehr guter Wasserlöslichkeit, wie Methanol oder Ethanol, zu Produkten mit niedrigerer mittlerer Teilchengröße, während die Hydrolyse in Gegenwart von n- oder i-Propanol Produkte mit größerem mittlerem Teilchendurchmesser ergibt.

Die für den Einsatz dieser Polymerisate im Hygienebereich erforderlichen mittleren Teilchendurchmesser im Bereich von 100 bis 850 µm können erfindungsgemäß ohne Mahlen und Sieben erhalten werden. Das Verfahren kann so durchgeführt werden, wobei der Anteil der Polymerisatteilchen mit einem Teilchendurchmesser <100 µm zwischen 5 und 15 Gew.-% liegt.

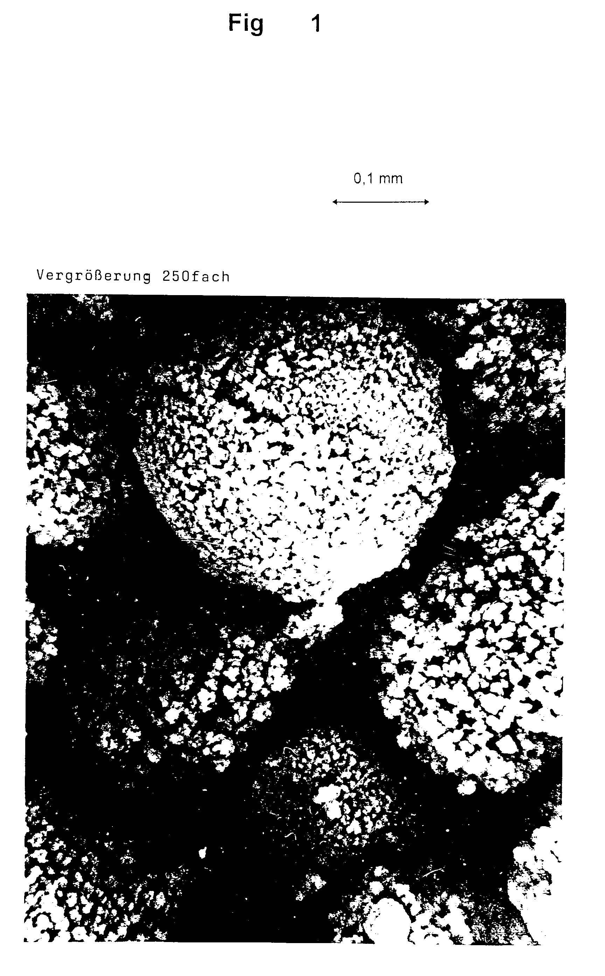

Im Verlauf der Hydrolyse gehen die feinteiligen Emulsionen auf Basis von Polyacrylnitril in eine leicht rührbare Suspension über, die nach Reaktionsende ohne Schwierigkeiten filtriert und getrocknet werden kann. Abbildung 1 zeigt eine elektronenmikroskopische Aufnahme der auf die beschriebene Weise erhaltenen superabsorbierenden Polymerisate. Man erkennt deutlich diskrete Teilchen mit kugelförmiger Morphologie und stark strukturierter Oberfläche.

Die nach dem beanspruchten Verfahren erhaltenen Produkte weisen ausgezeichnete superabsorbierende Eigenschaften auf: So beträgt ihr freies Quellvermögen in Wasser bzw. 0,9 %iger NaCl-Lösung bis zu 700 g/g bzw. bis zu 60 g/g und die Zentrifugenkapazität (C.C.) bis zu 45 g/g.

Ein weiterer Vorteil dieser Produkte ist, daß sie im gequollenen Zustand eine hohe Gelfestigkeit aufweisen und die gequollenen, kugelförmigen Teilchen den Weitertransport zusätzlich aufgegebener Flüssigkeit fördern. Diese Eigenschaft ist besonders bei der Herstellung von Hygieneartikeln, wie z.B. Babywindeln, erwünscht, da durch den Einsatz solcher Produkte das sogenannte "Gel-Blocking" verhindert wird.

Durch nachfolgendes Erhitzen der erhaltenen superabsorbierenden Polymerisate auf 150 bis 250°C, bevorzugt 170 bis 210°C, für 2 bis 30 Minuten, bevorzugt 5 bis 15 Minuten, können deren bereits ausgezeichnete anwendungstechnische Eigenschaften nochmals deutlich verbessert werden. Dies betrifft besonders die Quellungskinetik, d.h. die Absorptionsgeschwindigkeit für Wasser und andere Flüssigkeiten, sowie die Gelfestigkeit der gequollenen Polymerisate und ihr Aufnahmevermögen für wäßrige Flüssigkeiten unter Druck.

Die superabsorbierenden Polymerisate eignen sich vorzüglich zum Einsatz bei der Herstellung von Hygieneartikeln, wie z.B. Babywindeln oder Inkontinenzprodukte, sowie zur Ummantelung von Elektrokabeln. Außerdem können die Produkte in der Landwirtschaft als wasserspeichernde Materialien eingesetzt werden.

### Beispiele

### Beispiel 1

In einem 2 l-Vierhalskolben, der mit Rückflußkühler, Thermometer, Tropftrichter und Rührer ausgerüstet ist, werden 358,8 g einer feinteiligen (mittlerer Teilchendurchmesser: 95 nm), wäßrigen Polymerisat-Emulsion mit der Zusammensetzung 99,25 Gew.-% Acrylnitril und 0,75 Gew.-% Divinylbenzol, die einen Feststoffgehalt von 22,16 % aufweist, sowie 100 g Ethanol unter Rühren vorgelegt. Der Reaktorinhalt wird auf 50°C aufgeheist. Danach wird ein Gemisch aus 115 g Ethanol und 90,4 g einer 45,0 Gew.-%igen wäßrigen NaOH-Lösung zugegeben. Demnach hat man zu Anfang ein Reaktionsgemisch mit folgender Zusammensetzung vorliegen: 12,0 Gew.-% Polyacrylnitril und 6,14 Gew.-% NaOH, wobei das Molverhältnis Polyacrylnitril zu NaOH 1:0,68 und das Gewichtsverhältnis von Wasser zu Ethanol 1:0,65 beträgt.

Unter Rühren und Überleiten von N₂ wird das Reaktionsgemisch auf 78 bis 79°C erhitzt. Nach Erreichen eines Hydrolysegrades von 58,5% - ermittelt durch quantitative Bestimmung des abgespaltenen Ammoniaks - wird auf 20°C abgekühlt. Nach dem Abkühlen wird ein Gemisch aus 450 g Ethanol und 53,5 ml einer 5 molaren Ameisensäurelösung zur Neutralisation der nicht verbrauchten Natronlauge 15 Minuten in den Reaktor dosiert. Nach Abfiltrieren und Waschen mit 500 ml Ethanol sowie Trocknen im Vakuum bei 65°C erhält man 127 g eines farblosen Pulvers, das ohne Mahlen durch Siebung klassiert wird. Teilchengrößenverteilung: <100 µm: 12,5 Gew.-%; 100 - 850 µm: 87,5 Gew.-%.

### Thermische Behandlung:

Das nach Beispiel 1 erhaltene Polymerisat (mittlerer Teilchendurchmesser: 100 - 850 µm) wird in einem Umlufttrockenschrank ca. 15 Minuten bei einer Temperatur von 165°C gehalten.

### Bestimmung des Quellungsgrades:

250 mg des zu untersuchenden superabsorbierenden Polymerisats werden in ein 300 ml-Becherglas eingewogen und mit 250 bis 300 ml destilliertem Wasser bzw. mit 50 ml einer 0,9 Gew.-%igen NaCl-Lösung übergossen und stehen gelassen.

Nach Erreichen des Gleichgewichtsquellungszustandes wird das erhaltene Gel über ein Filtertuch mit der Maschenweite 30 µm oder ein Papierfilter abfiltiert und ausgewogen. Der Quellungsgrad errechnet sich dann aus dem Verhältnis Auswaage:Einwaage in g/g. Jede Bestimmung wird dreimal durchgeführt. Die Messgenauigkeit beträgt ±5%.

Für das gemäß Beispiel 1 dargestellte Produkt ergibt sich nach der thermischen Behandlung ein Quellungsgrad von 505 g/g in destilliertem Wasser und von 56,8 g/g in 0,9%iger NaCl-Lösung.

### pH-Wert-Bestimmung:

Der pH-Wert des gemäß Beispiel 1 erhaltenen Produktes beträgt in 0,9%iger NaCl-Lösung 5,7.

### Bestimmung des wasserlöslichen Anteils (WLA):

0,5 g des superabsorbierenden Polymerisats werden in 500 ml entionisiertes Wasser gegeben und 16 Stunden bei 20°C gerührt. Nach Filtrieren des Gels erhält man aus der Bestimmung des Feststoffgehaltes im Filtrat und Waschwasser den WLA. Dieser beträgt im Falle des nach Beispiel 1 erhaltenen Produktes 7,2 Gew.-%.

### Messung der Zentrifugenkapazität (C.C.):

Die Zentrifugenkapazität (C.C.) wird nach der Teebeutelmethode bestimmt und als Mittelwert aus 3 Messungen angegeben: 660 mg superabsorbierendes Polymerisat (SAP) werden in einen Teebeutel eingeschweißt und 20 Minuten lang in eine 0,9%ige wäßrige NaCl-Lösung eingetaucht. Anschließend wird der Teebeutel in einer Zentrifuge bei 1400 UpM 5 Minuten lang geschleudert und ausgewogen. Zur Ermittlung des Blindwertes läßt man einen leeren Teebeutel mitlaufen.
**Zentrifugenkapazität = (Auswaage - Blindwert) - Einwaage/Einwaage [g/g]**
Die Zentrifugenkapazitat des nach Beispiel 1 erhaltenen Produktes beträgt 42,1 g/g.

### Messung der Gelfestigkeit:

Verwendete Apparatur: Stevens L.F.R.A. Texture Analyzer.

Messprinzip: eine zylindrische oder konische Prüfsonde wird mit einer vorgewählten Geschwindigkeit und Meßstrecke in das Gelprüfmuster gedrückt. Die daraus resultierende Kraft in g wird gemessen und digital angezeigt.

Durchführung der Messungen: 1 g der zu untersuchenden Probe wird in 170 ml entionisiertem Wasser 2 Stunden lang gequollen. Dananch wird das Gel in ein 150 ml-Becherglas überführt. Nach Temperierung der Probe bei 20°C wird das Messgerät auf eine Geschwindigkeit von 1 mm/sec und einen Penetrationsweg von 10 mm sowie auf das Prüfprogramm "normal" eingestellt. Als Prüfsonde verwendet man die Zylinderprüfsonde mit der Bezeichnung TA3, welche einen Durchmesser von 1 Zoll aufweist. Mit jeder Probe werden mindestens zwei Messungen durchgeführt.

Die Gelfestigkeit des gemäß Beispiel 1 erhaltenen Produktes beträgt 106 g.

### Beispiel 2 bis 12:

Die Hydrolysebedingungen der nach den Beispielen 2 bis 12 hergestellten Proben sind in Tabelle 1 zusammengefasst.

Die Eigenschaften der bei diesen Hydrolyseversuchen erhaltenen superabsorbierenden Polymerisaten, deren Teilchengrößenverteilungen im Bereich von 100 bis 850 µm liegen, gehen aus Tabelle 2 hervor.

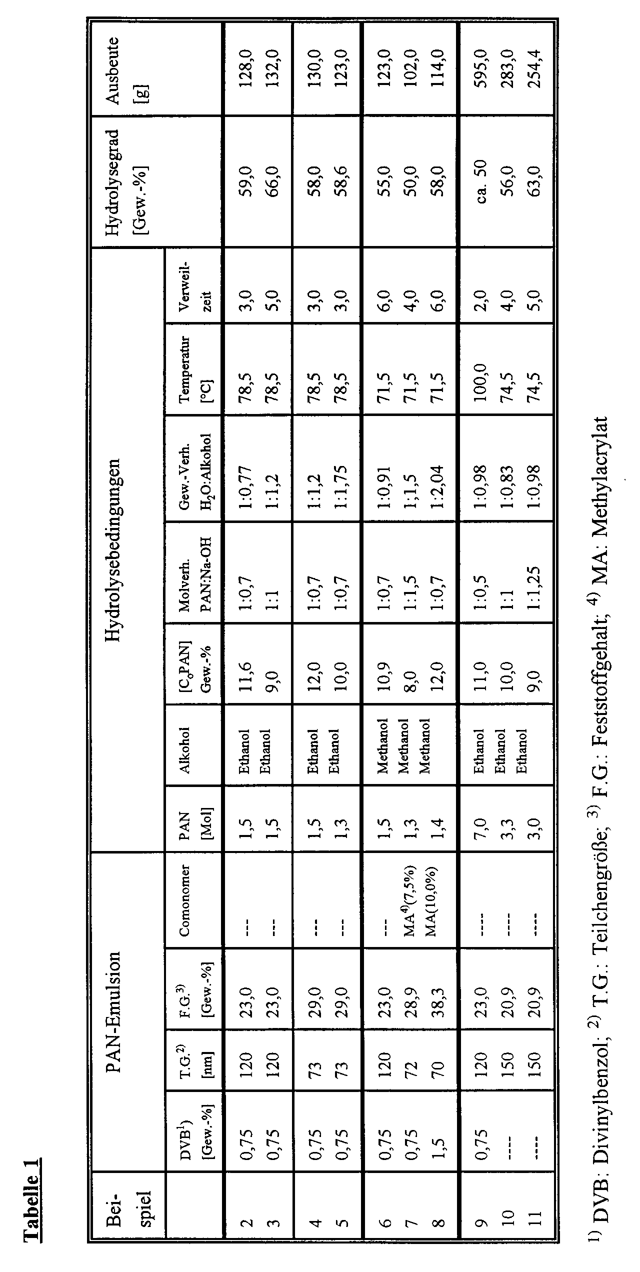

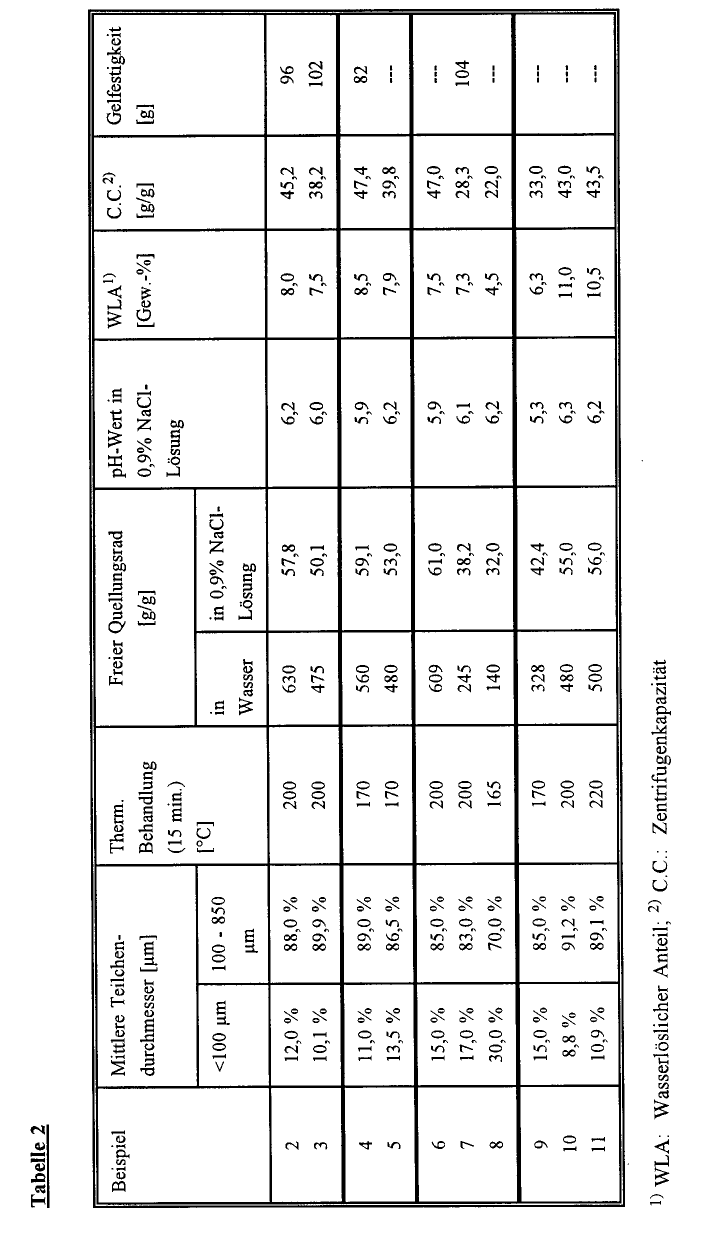

### Beispiele 2 und 3:

Als Ausgangsprodukt für die Hydrolysereaktion wurde eine mit 0,75 Gew.-% Divinylbenzol (DVB) vernetzte PAN-Emulsion mit einem mittleren Teilchendurchmesser von 120 nm und einem Feststoffgehalt von 23,0 Gew.-% eingesetzt. Die Hydrolyse wurde in einem Wasser/Ethanol-Gemisch gemäß der in Beispiel 1 beschriebenen Methode mit einem ansteigenden Molverhältnis von NaOH zu PAN und einem ansteigenden Gewichtsverhältnis von Ethanol zu Wasser durchgeführt.

### Beispiele 4 und 5:

Für diese Beispiele wurde als Ausgangsprodukt zur Hydrolyse eine mit 0,75 Gew.-% DVB vernetzte PAN-Emulsion mit einem mittleren Teilchendurchmesser von 73 nm und einem Feststoffgehalt von 29,0 Gew.-% eingesetzt. Die Hydrolyse wurde in einem Wasser/-Ethanol-Gemisch entsprechend der in Beispiel 1 angegebenen Methode durchgeführt. Dabei betrug das Molverhältnis von PAN zu NaOH ebenfalls 1:0,7, während die Gewichtsverhältnisse Wasser:Alkohol variiert wurden.

### Beispiele 6 bis 8:

Die Hydrolysereaktionen wurden in einem Wasser/Methanol-Gemisch analog zu der in Beispiel 1 beschriebenen Versuchsführung durchgeführt. Als Ausgangsprodukte dienten vernetzte PAN-Emulsionen sowohl als Homopolymerisat (S. Beispiel 6) sowie als Copolymerisate mit Methylacrylat (Beispiele 7 und 8) als Comonomer.

### Beispiel 9:

In einem 6 l-Autoklaven werden 1613 g (7,0 Mol) einer mit 0,75 Gew.-% DVB vernetzten PAN-Emulsion mit dem mittleren Teilchendurchmesser 120 nm und einem Feststoffgehalt von 23,0 Gew.-% und 450 g Ethanol unter Rühren vorgelegt. Nach kurzzeitigem Evakuieren des Autoklaven (ca. 10 mbar) wird der Reaktorinhalt auf 50°C erwärmt. Danach wird ein Gemisch aus 1000 g Ethanol und 311 g (3,5 Mol) einer 45 Gew.-%igen wäßrigen NaOH-Lösung unter Vakuum innerhalb von 45 Minuten zudosiert.

Zu Beginn der Reaktion hat das Reaktionsgemisch folgende Zusammensetzung: 11,0 Gew.-% Polyacrylnitril und 4,15 Gew.-% NaOH, womit das Molverhältnis Polyacrylnitril zu NaOH 1:0,5 und das Gewichtsverhältnis Wasser zu Ethanol 1:0,98 beträgt.

Der Reaktorinhalt wird auf 100°C erhitzt und 2 Stunden bei dieser Temperatur gehalten. Nach dem Abkühlen auf 20°C wird der Reaktor entspannt und das freigesetzte Ammoniak durch Einleiten in 1500 ml einer 20%-igen Schwefelsäure-Lösung absorbiert. Unter Rühren werden nun 790 g Ethanol in den Reaktor während 30 bis 40 Minuten dosiert. Der im Verlauf der Reaktion ausgefallene pulverförmige Niederschlag wird als Suspension durch das Bodenventil abgelassen. Die Neutralisation der Suspension erfolgt in 2,5 l eines Wasser/Ethanol-Gemischs (Gewichtsverhältnis Wasser:Ethanol = 1:3) mit 260 ml einer 5 molaren Ameisensäurelösung unter Rühren. Nach Abfiltrieren und Waschen mit 3 l Ethanol und anschließendem Trocknen im Vakuum bei 65°C erhält man ca. 595 g eines Pulvers, das ohne Mahlen durch Siebung klassiert wird.

Die Teilchengrößenverteilung und die Eigenschaften dieses superabsorbierenden Polymerisats sind in Tabelle 2 wiedergegeben.

### Beispiele 10 und 11:

Bei den Beispielen 10 und 11 wurde die Hydrolyse gleich wie in Beispiel 1 angegeben durchgeführt. Als Ausgangsprodukt für die Hydrolyse diente eine unvernetzte Emulsion mit einem [η]-Wert von 7,8 dl/g, einem mittleren Teilchendurchmesser von 150 nm und einem Feststoffgehalt von 20,9 Gew.-%.

## Patentansprüche

1. Superabsorbierendes Polymerisat in Form präformierter kugelförmiger Teilchen, erhältlich durch alkalische Hydrolyse von wäßrigen Emulsionen vernetzter und/oder unvernetzter Homo- und/oder Copolymerisate des Acrylnitrils in Wasser/Alkohol-Gemischen.

2. Superabsorbierendes Polymerisat gemäß Anspruch 1, worin die Homo- und/oder Copolymerisate des Acrylnitrils nach Beendigung der Hydrolyse 30 bis 60 Mol-% Carboxylatgruppen, 20 bis 60 Mol-% Carbonamidgruppen und 10 bis 20 Mol-% Nitrilgruppen enthalten.

3. Verfahren zur Herstellung von präformierten, kugelförmigen superabsorbierenden Polymerisaten, dadurch gekennzeichnet, daß man feinteilige, wäßrige Emulsionen von vernetzten und/oder unvernetzten Homo- und/oder Copolymerisaten des Acrylnitrils durch Umsetzung mit Alkalihydroxiden in Wasser/Alkohol-Gemischen partiell hydrolysiert und die im Verlauf der Reaktion ausgefallenen Pulver abtrennt, trocknet und gegebenenfalls kurze Zeit erhitzt.

4. Verfahren gemäß Anspruch 3, worin als Alkohole primäre, aliphatische Monoalkohole eingesetzt werden.

5. Verfahren gemäß Anspruch 3, worin als Alkohole Methanol, Ethanol, n-Propanol, i-Propanol, n-Butanol oder i-Butanol, eingesetzt werden.

6. Verfahren gemäß Anspruch 3, worin das Gewichtsverhältnis von Wasser zu Alkohol 10:1 bis 1:10 betragt.

7. Teilchen gemäß Anspruch 1 mit mittleren Teilchendurchmessern im Bereich von 100 bis 850 µm.

8. Polymerisat gemäß Anspruch 1, worin der Anteil der Teilchen mit einem Durchmesser <100µm zwischen 5 und 15 Gew.-% beträgt.

9. Superabsorbierende Teilchen nach Anspruch 1, worin diese einer thermischen Nachbehandlung von 2 bis 30 Minuten bei Temperaturen von 150 bis 250°C unterzogen werden.

10. Verwendung der gemäß Anspruch 1 erhältlichen superabsorbierenden Polymerisate in Hygieneprodukten, als wasserspeichernde Materialien in der Landwirtschaft oder bei der Ummantelung von Elektrokabeln.
